# EUROPÄISCHE PATENTSCHRIFT

(11) **EP 4 132 432 B1**
(45) Veröffentlichungstag und Bekanntmachung des Hinweises auf die Patenterteilung: **28.05.2025**
(21) Anmeldenummer: 21717379.8
(22) Anmeldetag: 01.04.2021
(51) Int. Cl.: A61F 2/68, A61F 2/72, A61F 2/70

(54) **AKTUIERBARE PROTHESENEINRICHTUNG, ELEKTRONISCHE DATENVERARBEITUNGSEINRICHTUNG UND VERFAHREN ZUM AKTUIEREN EINER PROTHESENEINRICHTUNG**
ACTUATABLE PROSTHESIS DEVICE, ELECTRONIC DATA PROCESSING DEVICE, AND METHOD FOR ACTUATING A PROSTHESIS DEVICE
DISPOSITIF DE PROTHÈSE ACTIONNABLE, DISPOSITIF DE TRAITEMENT DE DONNÉES ÉLECTRONIQUES ET PROCÉDÉ D'ACTIONNEMENT D'UN DISPOSITIF DE PROTHÈSE

(30) Priorität: 06.04.2020 DE 102020109509
(43) Veröffentlichungstag der Anmeldung: 15.02.2023
(73) Patentinhaber: Otto Bock Healthcare Products GmbH, 1110 Wien (AT)
(72) Erfinder: AMSÜSS, Sebastian, 1180 Wien (AT); SAGMEISTER, Luis, 2823 Pitten (AT); STEININGER, Johannes, 2154 Kleinbaumgarten (Gaubitsch) (AT); BALL, Carina, Doris, 1020 Wien (AT); MAYRHOFER, Patrick, 1110 Wien (AT)
(74) Vertreter: Gramm, Lins & Partner Patent- und Rechtsanwälte PartGmbB
(86) Internationale Anmeldenummer: PCT/EP2021/058674
(87) Internationale Veröffentlichungsnummer: WO 2021/204690

(56) Entgegenhaltungen:
- DE-A1- 102009 056 466
- DE-B3- 102017 119 490
- US-A1- 2012 101 596
- US-A1- 2014 100 667
- US-A1- 2016 051 382

## Beschreibung

Die Erfindung betrifft eine aktuierbare Protheseneinrichtung, mit zumindest einem Antrieb, einer elektronischen Datenverarbeitungseinrichtung und zumindest einer Sensoranordnung zum Anordnen an einem Körperteil eines Prothesenträgers, wobei die zumindest eine Sensoranordnung eingerichtet ist zum Erfassen von Körpersignalmustern und die elektronische Datenverarbeitungseinrichtung eingerichtet ist, den zumindest einen Antrieb in Abhängigkeit eines erfassten Körpersignalmusters derart anzusteuern, dass die Protheseneinrichtung eine dem Körpersignalmuster zugeordnete Prothesenbewegung ausführt.

Gemäß einem weiteren Aspekt betrifft die Erfindung zudem eine elektronische Datenverarbeitungseinrichtung für eine solche Protheseneinrichtung.

Gemäß einem dritten Aspekt betrifft die Erfindung ein Verfahren zum Aktuieren einer solchen Protheseneinrichtung.

Aktuierbare Protheseneinrichtungen sind beispielsweise Prothesen der oberen oder unteren Extremitäten. Protheseneinrichtungen sind auch Prothesenteile, wie beispielsweise eine Prothesenhand oder ein Prothesenfuß. Moderne Protheseneinrichtungen werden üblicherweise mittels einer elektronischen Datenverarbeitungseinrichtung gesteuert. Hierzu ist der Protheseneinrichtung mindestens ein Antrieb zugeordnet, welcher über die elektronische Datenverarbeitungseinrichtung ansteuerbar ist. Um möglichst gezielte Bewegung ausführen zu können und diese durch einen Prothesenträger möglichst intuitiv und leicht auslösen lassen zu können, weist eine solche aktuierbare Protheseneinrichtung üblicherweise zumindest eine Sensoranordnung auf. Diese ist häufig an einem Körperteil des Prothesenträgers angeordnet, dem die Protheseneinrichtung zugeordnet ist.

Mittels der zumindest einen Sensoranordnung können dann beispielsweise willentliche Körpersignale des Prothesenträgers erfasst und an die elektronische Datenverarbeitungseinrichtung geleitet werden. In dieser sind dann den Körpersignalen einzelne Prothesenbewegungen zugeordnet, sodass die elektronische Datenverarbeitungseinrichtung den zumindest einen Antrieb derart ansteuern kann, dass die Protheseneinrichtung die dem Körpersignal zugeordnete Prothesenbewegung ausführt. Solche Körpersignale sind beispielsweise die Kontraktion bestimmter, einzelner Muskeln oder Muskelgruppen in bestimmter Intensität und/oder Frequenz oder auch Kokontraktionen.

Daneben ist es bekannt, sogenannte Körpersignalmuster mittels der zumindest einen Sensoranordnung zu erfassen. Bei solchen Körpersignalmustern handelt es sich um komplexe Signalmuster, zum Beispiel Abfolgen von Muskelkontraktionen des Amputationsstumpfes. Diese können von einem Prothesenträger gezielt trainiert werden und sich beispielsweise an einem Bewegungsmuster orientieren, welches der Prothesenträger im unversehrten Zustand zur Bewegung der mit der Prothese versorgten Extremität verwendet hat. Gerade bei Patienten, deren Amputation noch nicht lange zurückliegt, besteht eine gute Erinnerung an die notwendigen Muskelkontraktionen. So können beispielsweise radialamputierte Patienten eine Kontraktion der Unterarmmuskulatur ausüben, die im unversehrten Zustand mit einer Greifbewegung der Hand assoziiert war. Diese Körpersignalmuster werden dann bevorzugt mittels einer Sensoranordnung erfasst, in der elektronischen Datenverarbeitungseinrichtung und/oder einem dieser zugeordneten Speichermedium gespeichert und einer bestimmten Prothesenbewegung, in diesem Beispiel insbesondere einer solchen Greifbewegung, zugeordnet.

Wird eine bestimmte Bewegung oder ein bestimmtes Muskelkontraktionsmuster ausgeführt und mittels der Sensoranordnung als ein bestimmtes Körpersignalmuster erkannt, so wird über eine in der elektronischen Datenverarbeitungseinrichtung abgelegte Steuerung ein Befehl an den zumindest einen Antrieb übermittelt, eine bestimmte Verlagerung oder Verschwenkung einer Prothesenkomponente über einen bestimmten Weg in einer bestimmten Zeit auszuführen.

Problematisch ist hierbei jedoch, dass aufgrund deren Komplexität nur eine begrenzte Anzahl an solchen Körpersignalmustern von einem Prothesenträger erzeugt werden kann. Hierdurch ist entsprechend die Anzahl an zuordenbaren Prothesenbewegungen limitiert, was einer möglichst realitätsnahen Nachbildung des ersetzten Körperteils durch die Protheseneinrichtung entgegensteht. Selbst sehr gute und geübte Prothesenträger können selten mehr als acht Körpersignalmuster reproduzierbar erzeugen.

Die US 2014/100667 A1 betrifft ein Verfahren zur Steuerung mindestens eines angetriebenen Gelenks in einer Prothese. Zuerst werden myoelektrische Steuersignale vom Benutzer während eines nicht gewichtsbelastenden Modus für ein angetriebenes Gelenk empfangen. Dann wird eine Geschwindigkeitsreferenz für ein angetriebenes Gelenk auf der Grundlage der myoelektrischen Steuersignale erzeugt. Auf der Grundlage der Geschwindigkeitsreferenz wird ein Signal zur Betätigung eines angetriebenen Gelenks erzeugt.

Die DE 10 2009 056 466 A1 betrifft ein Verfahren zur adaptiven Steuerung und Regelung einer Prothese mit Willkürsteuerung zur nahezu naturgemäßen Bewegung einer Prothese. Zunächst werden an einem EMG-Array die Muskelaktivitäten des Benutzers gemessen. Anschließend wird im Zuge eines Klassifizierungsverfahrens, bei dem sowohl die aufgezeichnete Muskelaktivität als auch der aktuelle Bewegungszustand berücksichtigt werden, ein Willkürsignal bestimmt. Dieses Willkürsignal dient zur Regelung und Steuerung einer Aktuatorik einer Prothese.

Die DE 102017 119 490 B3 betrifft ein Prothesensystem mit zumindest zwei Sensoren und einer Steuerungseinrichtung, die mit den Sensoren gekoppelt ist.

Aktuatoren werden über die Steuerungseinrichtung aktiviert oder deaktiviert, um zumindest eine beweglich gelagerte Prothesenkomponente zu verlagern. In der Steuerungseinrichtung ist ein Standardprogramm hinterlegt, das jedem Sensor eine Aktuatoraktion unabhängig von der Dauer und/oder Intensität des Sensorsignals zuordnet. Damit kann eine Überprüfung der Funktionsfähigkeit des Prothesensystems durchgeführt werden.

Die US 2016/0051382 A1 betrifft ein Verfahren zum Betreiben einer Greifvorrichtung, bei dem mehrere parallele, bidirektionale Zustandsflusskarten verwendet werden, die jeweils eine Abfolge von Stellungen für mehrere Gelenke in der Greifvorrichtung definieren. Das Verfahren umfasst das Empfangen mindestens eines Steuersignals, das Bestimmen einer aktuellen Stellung der Greifvorrichtung innerhalb einer der mehreren Zustandsflusskarten, die derzeit für die Greifvorrichtung ausgewählt sind, und das selektive Betätigen der mehreren Gelenke, um die Sequenz von Stellungen zu durchlaufen, wobei eine Richtung zum Durchlaufen der Stellungsfolge auf Basis des mindestens einen Steuersignals ermittelt wird.

Die US 2016/0101596 A1 betrifft ein Verfahren zum Einrichten einer Steuerung einer orthopädietechnischen Einrichtung, die an einem Körperteil eines Patienten angelegt ist und mit Sensoren verbunden ist, die biometrische Daten eines Patienten aufnehmen und eine Steuerung der orthopädietechnischen Einrichtung. Zunächst wird eine Darstellung einer Betätigung einer Gliedmaße ausgegeben, um damit einen Patienten aufzufordern, diese Betätigung auszuführen. Biometrische Signalen, die von dem Patienten nach Aufforderung als willkürliche Reaktion erzeugt werden, werden erfasst und die erzeugten Signale werden der ausgeführten Betätigung zugeordnet. Anschließend wird die Signalzuordnung gespeichert.

Vor diesem Hintergrund ist es Aufgabe der vorliegenden Erfindung eine möglichst einfach aktuierbare Protheseneinrichtung vorzuschlagen, mit welcher eine größere Anzahl an Prothesenbewegungen möglich ist.

Die Erfindung löst diese Aufgabe durch eine aktuierbare Protheseneinrichtung mit den Merkmalen des Hauptanspruches, durch eine elektronische Datenverarbeitungseinrichtung für eine solche Protheseneinrichtung sowie ein Verfahren zum Aktuieren einer solchen Protheseneinrichtung. Vorteilhafte Ausgestaltungen und Weiterbildungen der Erfindung sind in den Unteransprüchen, der Beschreibung und den Figuren offenbart.

Die aktuierbare Protheseneinrichtung, mit zumindest einem Antrieb, einer elektronischen Datenverarbeitungseinrichtung und zumindest einer Sensoranordnung zum Anordnen an einem Körperteil eines Prothesenträgers, wobei die zumindest eine Sensoranordnung eingerichtet ist zum Erfassen von Körpersignalmustern und die elektronische Datenverarbeitungseinrichtung eingerichtet ist, den zumindest einen Antrieb in Abhängigkeit eines erfassten Körpersignalmusters derart anzusteuern, dass die Protheseneinrichtung eine dem Körpersignalmuster zugeordnete Prothesenbewegung ausführt, sieht vor, dass in der elektronischen Datenverarbeitungseinrichtung zumindest zwei Sets aus Körpersignalmustern und diesen jeweils zugeordneten Prothesenbewegungen hinterlegt sind, und den Sets zumindest ein Triggersignal zugeordnet ist, das zwischen den Sets umschaltet, wobei in mehreren Sets, bevorzugt in jedem Set, einem oder mehreren Körpersignalmuster/nn dieselben Prothesenbewegungen zugeordnet sind.

Dadurch, dass in der elektronischen Datenverarbeitungseinrichtung zumindest zwei Sets aus Körpersignalmustern und diesen jeweils zugeordneten Prothesenbewegungen hinterlegt sind, kann die Anzahl der durch die aktuierbare Protheseneinrichtung ausführbaren Prothesenbewegungen auf einfache Weise erhöht werden.

Im einfachsten Fall enthält die elektronische Datenverarbeitungseinrichtung genau zwei Sets und genau ein Triggersignal. Um nun eine bestimmte Prothesenbewegung auszuführen, führt der Prothesenträger eine Bewegung, insbesondere ein Muskelkontraktionsmuster aus, welches mittels der Sensoranordnung erfasst wird und von der elektronischen Datenverarbeitungseinrichtung als ein bestimmtes Körpersignalmuster erkannt wird. Die elektronische Datenverarbeitungseinrichtung prüft dann zunächst, welches der beiden Sets ausgewählt ist und steuert den Antrieb entsprechend der Prothesenbewegung an, die dem Körpersignalmuster in dem ausgewählten Set zugeordnet ist. Um beispielsweise von dem ersten Set zu dem zweiten Set umzuschalten, muss das Triggersignal einmal ausgelöst werden. Um von dem zweiten Set wieder zu dem ersten Set umzuschalten, wird das Triggersignal erneut ausgelöst. Wenn der Prothesenträger mittels des Triggersignals das gewünschte Set ausgewählt hat, führt er die Bewegung oder das Muskelkontraktionsmuster aus, die oder das dem Körpersignalmuster für die gewünschte Prothesenbewegung entspricht. Eine solche sequentielle Umschaltung bietet den Vorteil, dass der Prothesenträger nur ein Triggersignal erlernen und/oder sich merken muss.

In mehreren Sets, bevorzugt in jedem Set, sind einem oder mehreren Körpersignalmustern dieselben Prothesenbewegungen zugeordnet. So sind beispielsweise in einem ersten Set den Körpersignalmustern 1 bis 4 die Prothesenbewegungen 1 bis 4 zugeordnet. In dem zweiten Set ist dem Körpersignalmuster 1 erneut Prothesenbewegung 1 zugeordnet, den Körpersignalmustern 2 bis 4 jedoch die Prothesenbewegungen 5 bis 7. Dies ist insbesondere dann vorteilhaft, wenn eine Prothesenbewegung, in dem Beispiel die Prothesenbewegung 1, eine besonders häufig auszuführende Prothesenbewegung ist. Diese kann dann, unabhängig davon welches Set gerade anzuwenden ist, mit demselben Körpersignalmusters ausgelöst werden. Dies vermeidet unnötig häufiges Umschalten mittels des zumindest einen Triggersignals und erhöht den Anwendungskomfort.

Gemäß einer Weiterbildung ist jedem Set jeweils ein unterschiedliches Triggersignal zugeordnet, welches dann entsprechend genau für dieses Set kodiert. So kann beispielsweise einem ersten Set ein erstes Triggersignal, einem zweiten Set ein zweites Triggersignal und einem dritten Set ein drittes Triggersignal zugeordnet sein.

Durch Auslösen des jeweiligen Triggersignals wird direkt auf das zugehörige Set umgeschaltet. Dies ist beispielswiese bei zwei Sets vorteilhaft, da der Prothesenträger zum Auswählen eines bestimmten Sets nicht wissen muss, welches Set momentan ausgewählt ist. Besonders vorteilhaft ist diese Ausführungsform, wenn mehr als zwei Sets vorhanden sind, da insbesondere ein aufwendiges sequentielles Umschalten entfällt.

Die Protheseneinrichtung weist in einer Weiterbildung eine Anzeigevorrichtung auf, die das momentan ausgewählte Set anzeigt. Dies ist beispielsweise ein Display oder eine oder mehrere Leuchtdioden.

Bevorzugt ist eines der Sets als Standardset hinterlegt. Sofern beispielsweise nicht mittels eines vom Prothesenträger ausgelösten Triggersignals von einem anderen Set auf dieses umgeschaltet wurde, erfolgt die Einstellung auf das Standardset automatisch. Beispielsweise erfolgt dies automatisch nach einer vorgegebenen Zeitspanne von beispielsweise zumindest 10 Sekunden, insbesondere zumindest 30 Sekunden. Vorzugsweise ist diese Zeitspanne individuell einstellbar. Auf diese Weise ist es nicht notwendig, dass sich der Prothesenträger das momentan ausgewählte Set merken muss oder dass eine Anzeigevorrichtung vorhanden ist, die ihm das momentan ausgewählte Set anzeigt. Wenn der Prothesenträger die Protheseneinrichtung eine Zeit lang keine Prothesenbewegung hat ausführen lassen, weiß er, dass er sich stets im ersten Set befindet. Es ist jedoch möglich, dass zusätzlich zu einer solchen zeitgesteuerten Umschaltung auf das Standardset eine Anzeigevorrichtung für das momentan ausgewählte Set vorhanden ist. Alternativ wird das Standardset zunächst nur beim Einschalten ausgewählt, wird anschließend ein Triggersignal erzeugt und von dem Standardset auf ein erstes oder zweites Set umgeschaltet, können diese auch in dem ausgewählten Zustand verbleiben.

Bevorzugt ist in den Sets zumindest ein Körpersignalmuster identisch enthalten, wobei dem zumindest einen identisch enthaltenen Körpersignalmuster in den Sets jeweils unterschiedliche Prothesenbewegungen zugeordnet sind.

Dadurch, dass zumindest ein Körpersignalmuster in den Sets identisch enthalten ist, ist gewährleistet, dass dieses zumindest eine Körpersignalmuster doppelt belegt ist, also dasselbe Körpersignalmuster für mehr als eine Prothesenbewegung kodiert. Durch Auswahl eines bestimmten Sets mittels des zumindest einen Triggersignals kann entsprechend die Prothesenbewegung ausgewählt werden, die über das bestimmte Körpersignalmuster ausgeführt werden soll.

Es ist zudem möglich, dass mehr als nur ein Körpersignalmuster identisch in den Sets enthalten ist. So können beispielsweise zwei, drei, vier, fünf, sechs, sieben oder acht der in den Sets enthaltenen Körpersignalmuster identisch sein.

Bevorzugt enthält jedes Set die identischen Körpersignalmuster. Mit anderen Worten enthält jedes Set genau die gleichen Körpersignalmuster, wobei jedem Körpersignalmuster in den unterschiedlichen Sets bevorzugt jeweils unterschiedliche Prothesenbewegungen zugeordnet sind. Beispielsweise sind in einem ersten Set den Körpersignalmustern 1 bis 4 die Prothesenbewegungen 1 bis 4 zugeordnet. In dem zweiten Set sind den Körpersignalmustern 1 bis 4 dann die Prothesenbewegungen 5 bis 8 zugeordnet. Auf diese Weise ist es möglich, bei nur vier Körpersignalmustern acht verschiedene Prothesenbewegungen auszuführen.

Gemäß einer Weiterbildung der Erfindung sind in der elektronischen Datenverarbeitungseinrichtung zumindest drei, weiter bevorzugt zumindest vier Sets hinterlegt, sodass die Anzahl an Prothesenbewegungen stark erhöht wird, ohne die Anforderungen an den Prothesenträger, nämlich weitere Körpersignalmuster zu erlernen, erhöht werden müssen.

Bevorzugt ist das zumindest eine Triggersignal ein festgelegtes Körpersignal, das von den in den Sets hinterlegten Körpersignalmustern abweicht. Bevorzugt handelt es sich folglich bei dem zumindest einen Triggersignal nicht um ein eigenständiges Körpersignalmuster, sondern um ein Körpersignal wie beispielsweise eine Kokontraktion eines bestimmten Muskels oder einer bestimmten Muskelpartie. Dies ist insbesondere vorteilhaft, um nicht eines der wenigen verfügbaren Körpersignalmuster mit dem zumindest einen Triggersignal zu belegen, sondern dieses für eine oder mehrere Prothesenbewegungen freizuhalten. Bei unterschiedlichen Triggersignalen, die beispielsweise jeweils einem bestimmten Set zugeordnet sind, können die jeweiligen Triggersignale der gleichen Kategorie angehören, also beispielsweise alle unterschiedliche Kokontraktionen sein. Es ist jedoch ebenfalls möglich, dass die unterschiedlichen Triggersignale unterschiedlichen Kategorien angehören, beispielsweise Kokontraktionen, Kontraktionsmuster, Kontraktionsdauer und/oder Kontraktionsintensität.

Bevorzugt umfasst jedes Set zumindest vier Körpersignalmuster, insbesondere zumindest sechs Körpersignalmuster.

Die Anzahl an Körpersignalmustern pro Set kann beispielsweise in Abhängigkeit des Erfahrungsgrades eines Prothesenträgers, für den die jeweilige Protheseneinrichtung vorgesehen ist, bestimmt werden. Bei unerfahrenen und ungeübten Prothesenträgern kann es beispielsweise vorteilhaft sein, pro Set nur genau zwei, genau drei oder genau vier Körpersignalmuster vorzusehen. Bei manchen, insbesondere erfahreneren Trägern kann es hingegen vorteilhaft sein, wenn genau fünf, genau sechs, genau sieben oder genau acht Körpersignalmuster pro Set hinterlegt sind. Bevorzugt weist jedes Set nicht mehr als zwölf, besonders bevorzugt nicht mehr als 10 Körpersignalmuster auf, da dies die allermeisten Prothesennutzer nicht mit der notwendigen Präzision und Unterscheidbarkeit hervorbringen können.

Bevorzugt weist die zumindest eine Sensoranordnung mehrere Elektrodenpaare zum Erfassen der Körpersignalmuster und insbesondere ein Elektrodenpaar zum Erfassen des zumindest einen Triggersignals auf.

Die zumindest eine Sensoranordnung weist zum Erfassen der Körpersignalmuster bevorzugt eine Mehrzahl von Elektrodenpaaren, beispielsweise zumindest zwei, besonders bevorzugt zumindest vier, insbesondere genau vier Elektrodenpaare auf. Es ist möglich, dass ein weiteres Elektrodenpaar zum Erfassen des zumindest einen Triggersignals vorhanden ist. Es ist jedoch ebenfalls möglich, dass eines der Elektrodenpaare zum Erfassen der Körpersignalmuster gleichzeitig das Elektrodenpaar zum Erfassen des zumindest einen Triggersignals ist. Die Sensoranordnung oder das Elektrodenpaar zum Erfassen des zumindest einen Triggersignals kann auch insbesondere an einer Gliedmaße oder an einem Körperteil angeordnet sein, das nicht mit der Prothese oder Prothesenkomponente in funktionaler Verbindung steht.

Die Elektroden der zumindest einen Sensoranordnung sind bevorzugt gleichmäßig oder im Wesentlichen gleichmäßig zueinander beabstandet um die Gliedmaße, den Gliedmaßenstumpf oder den Amputationsstumpf herum angeordnet, bei sechs Elektroden beispielsweise in einem Abstand von 60° oder ungefähr 60° zueinander, bei acht Elektroden in einem Abstand von 45° oder ungefähr 45° zueinander und bei vier Elektroden in einem Abstand von 90° oder ungefähr 90° zueinander.

Die Anzahl der notwendigen Elektroden oder Elektrodenpaare hängt von der Komplexität der jeweiligen Protheseneinrichtungen und der von dieser auszuführenden Bewegungen ab. Für komplexe prothetische Versorgungen, auf die sich diese Erfindung insbesondere bezieht, beispielsweise mit einem angetriebenen Prothesenellenbogengelenk, einer Prothesenhand mit einzeln verlagerbaren Prothesenfingern und/oder einem flektierbaren und/oder rotierbaren Handgelenk, sind teilweise mehr als acht Elektrodenpaare notwendig.

Die Art der Versorgung und die Anzahl der Elektrodenpaare hängen auch von der Fähigkeit des jeweiligen Trägers ab, bestimmte komplexe Körpersignalmuster zu erzeugen.

Bevorzugt sind die Sets mittels eines Eingabegeräts zumindest teilweise veränderbar. Hierzu kann beispielsweise ein Computer drahtlos oder über ein Kabel mit der elektronischen Datenverarbeitungseinrichtung verbunden werden und die in dieser hinterlegten Sets verändern. Gemäß einer Weiterbildung ist dies über eine App möglich, die beispielsweise auf einem Smartphone des jeweiligen Prothesenträgers oder eines Orthopädietechnikers hinterlegt ist.

Dass die Sets veränderlich sind bedeutet beispielsweise, dass eine Anzahl an pro Set hinterlegten Körpersignalmustern veränderlich ist und/oder die hinterlegten Prothesenbewegungen und/oder die Zuordnung einer bestimmten Prothesenbewegung zu einem bestimmten Körpersignalmuster veränderlich sind. Ebenfalls können ergänzend oder alternativ die Körpersignalmuster selbst, also insbesondere welche Muskeln oder Muskelpartien für ein bestimmtes Körpersignalmuster wie zu kontrahieren sind oder die Vorgaben und Parameter dazu, eingestellt bzw. verändert werden.

Über die zumindest eine Sensoranordnung ist es bevorzugt möglich, elektromyografische Signale aufzunehmen, welche dann hinsichtlich ihrer Signalqualität ausgewertet werden können. Unter Signalqualität wird unter anderem die Trennbarkeit der Signale verstanden, insbesondere die Gestalt oder der Verlauf des Signals oder der Ort, an dem das Signal aufgenommen wird. Weiterhin ist die Wiederholbarkeit des Signals ein Qualitätskriterium. Eine hohe Signalqualität ist erreicht, wenn das Signal immer gleich aussieht, wenn ein Patient also immer das gleiche Signal erzeugen kann. Auch die Aufrechterhaltbarkeit des Signals ist für die Signalqualität relevant. Wenn das Signal für einen längeren Zeitraum erzeugt und abgenommen werden kann ist dies besser als nur ein kurz andauerndes Signal, das ggf. als eine Störgröße aufgefasst und nicht berücksichtigt werden könnte. Dazu wird die Patienten mit der angelegten zumindest einen Sensoranordnung aufgefordert, Muskelaktivitäten auszuführen, beispielsweise diejenige Tätigkeit auszuführen, die die Protheseneinrichtung ausführen soll, beispielsweise Hand öffnen, Hand schließen, Handgelenk drehen oder die Hand flektieren. Selbstverständlich kann diese Tätigkeit nicht unmittelbar von den Patienten ausgeführt werden, da die durch die Protheseneinrichtung ersetzte Gliedmaße fehlt, jedoch können die verbliebenen Muskeln teilweise wie vor dem Verlust der Gliedmaße aktiviert werden. Dadurch entstehen elektrische Potentiale, die durch die zumindest eine Sensoranordnung aufgenommen und zu der elektronischen Datenverarbeitungseinrichtung übermittelt werden. Anhand beispielsweise der Signaldauer, der Signalintensität, der Flankensteigung oder auch einer Signalfrequenz bei Muskelkontraktionen ist es möglich festzulegen, ob eine konkrete Bewegung oder ein konkretes Muskelkontraktionsmuster geeignet ist, um als Körpersignalmuster verwendet zu werden.

Bevorzugt ist es möglich, dass über das Eingabegerät, insbesondere die App, einem Prothesenträger angezeigt wird, welche Sensoranordnung gerade welches Signal erfasst. Insbesondere wird zusätzlich die Signalqualität des Signals visuell dargestellt. Hierdurch kann der Prothesenträger bestimmte Muskelkontraktionsmuster ausführen und sich mittels des Eingabegeräts anzeigen lassen, was die zumindest eine Sensoranordnung hiervon erkannt hat. Hierdurch ist es insbesondere möglich, dass der Prothesenträger die Ausführung der Körpersignalmuster trainiert oder die Körpersignalmuster gegebenenfalls an die real erzielten Sensorwerte angepasst werden können.

Gemäß einem weiteren Aspekt löst die Erfindung die gestellte Aufgabe durch eine elektronische Datenverarbeitungseinrichtung für eine solche Protheseneinrichtung, wobei in der elektronischen Datenverarbeitungseinrichtung zumindest zwei Sets aus Körpersignalmustern und diesen jeweils zugeordneten Prothesen hinterlegt sind und den Sets zumindest ein Triggersignal zugeordnet ist, das zwischen den Sets umschaltet wobei unabhängig von dem Set zumindest einem Körpersignalmuster dieselbe Prothesenbewegung zugeordnet ist.

Gemäß einem dritten Aspekt löst die Erfindung die gestellte Aufgabe durch ein Verfahren zum Aktuieren einer Protheseneinrichtung mit den Schritten: a) Auswählen eines anzuwendenden Sets aus den zumindest zwei in der elektronischen Datenverarbeitungseinrichtung hinterlegten Sets durch Ausüben des zumindest einen Triggersignals, b) Erfassen eines Körpersignalmusters des Prothesenträgers mittels der zumindest einen Sensoranordnung, und c) Aktuieren der Protheseneinrichtung mittels des zumindest einen Antriebs in Abhängigkeit des erfassten Körpersignalmusters und des ausgewählten Sets, sodass die Protheseneinrichtung die Prothesenbewegung ausführt, die dem erfassten Körpersignalmuster in dem ausgewählten Set zugeordnet ist, wobei unabhängig von dem ausgewählten Set bei zumindest einem Körpersignalmuster dieselbe Prothesenbewegung ausgeführt wird.

Gemäß einer Weiterbildung weist Schritt c) folgende Schritte auf: Leiten eines für das erfasste Körpersignalmuster kodierenden Sensorsignals von der Sensoranordnung zu der elektronischen Datenverarbeitungseinrichtung, Auswerten des Sensorsignals in der elektronischen Datenverarbeitungseinrichtung, Bestimmen einer auszuführenden Prothesenbewegung in Abhängigkeit des Sensorsignals und des ausgewählten Sets und Erzeugen eines für die auszuführende Prothesenbewegung kodierenden Steuersignals, Leiten des Steuersignals an den zumindest einen Antrieb der Protheseneinrichtung, und Betreiben des zumindest einen Antriebs in Abhängigkeit des Steuersignals, sodass die Protheseneinrichtung die auszuführende Prothesenbewegung ausführt.

Das Sensorsignal oder die Sensorsignale und das Steuersignal werden beispielswiese mittels eines Datenkabels oder drahtlos zu der elektronischen Datenverarbeitungseinrichtung geleitet.

Im Folgenden wird die Erfindung anhand der beigefügten Figuren näher erläutert, dabei zeigt
- Fig. 1: eine Visualisierung einer Ausführungsform der elektronischen Datenverarbeitungseinrichtung mit darin hinterlegten Körpersignalmustern und diesen zugeordneten Prothesenbewegungen,
- Fig. 2: ein schematisches Ablaufdiagramm einer Ausführungsform des Verfahrens,
- Fig. 3: eine schematische Darstellung einer Ausführungsform der aktuierbaren Protheseneinrichtung in Form einer Prothese für eine obere Extremität, und
- Fig. 4: eine schematische Darstellung einer Ausführungsform der aktuierbaren Protheseneinrichtung in Form einer Prothese für eine untere Extremität.

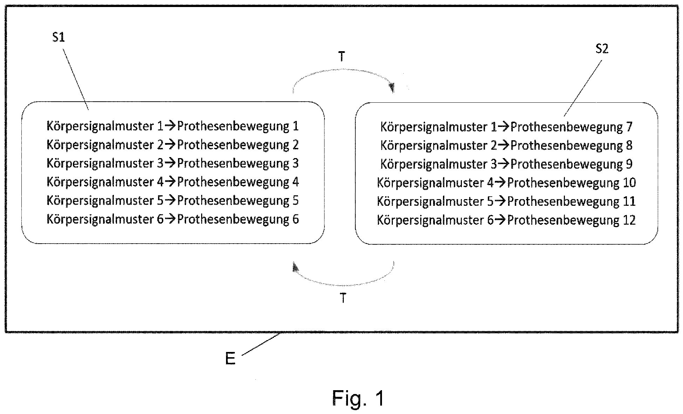

In Figur 1 ist eine elektronische Datenverarbeitungseinrichtung E angedeutet, in der ein erstes Set S1 und ein zweites Set S2 hinterlegt sind. Die Sets S1, S2 weisen jeweils sechs Körpersignalmuster auf. Die Körpersignalmuster sind in beiden Sets S1, S2 vollständig identisch, nämlich jeweils die Körpersignalmuster 1 bis 6. Jedem Körpersignalmuster ist eine Prothesenbewegung zugeordnet. Den Körpersignalmustern 1 bis 6 sind im ersten Set S1 die Prothesenbewegungen 1 bis 6 zugeordnet. In dem zweiten Set S2 sind denselben Körpersignalmustern 1 bis 6 andere Prothesenbewegungen zugeordnet, nämlich die Prothesenbewegungen 7 bis 12.

Durch diese Aufteilung ist es möglich, mit nur sechs verschiedenen Körpersignalmustern zwölf verschiedene Prothesenbewegungen auslösen zu können.

In der in Figur 1 dargestellten Ausführungsform ist nur genau ein Triggersignal T vorhanden. Um zwischen den Sets S1, S2 umzuschalten, muss der Prothesenträger das Triggersignal T ausführen oder erzeugen. Dieses ist beispielsweise eine Kokontraktion eines bestimmten Muskels oder einer bestimmten Muskelpartie. Mittels dieses Triggersignals T wird dann zwischen den Sets S1, S2 umgeschaltet. Gemäß einer weiteren Ausführungsform ist dem ersten Set S1 ein erstes Triggersignal T1 und dem zweiten Set S2 ein zweites Triggersignal T2 zugeordnet. Das erste Triggersignal T1 unterscheidet sich dabei von dem zweiten Triggersignal T2. Durch Ausüben oder Erzeugen des ersten Triggersignals T1 wird stets auf das erste Set S1 umgeschaltet und durch Ausüben oder Ausführen des zweiten Triggersignals T2 stets auf das zweite Set S2.

Bevorzugt ist einem der beiden Sets S1 oder S2 ein weiteres, zeitabhängiges Umschaltsignal zugeordnet. So schaltet die elektronische Datenverarbeitungseinrichtung E beispielsweise nach 10 Sekunden, 20 Sekunden oder 30 Sekunden automatisch von Set S2 zu Set S1, wenn dies nicht von dem Träger der Protheseneinrichtung durch Auslösen des Triggersignals T geschehen ist. In diesem Beispiel bildet dann das Set S1 das Standardset.

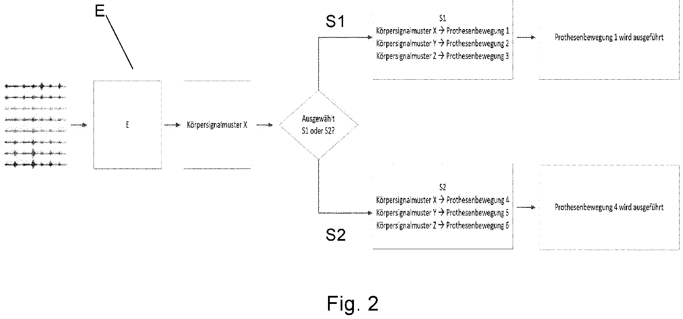

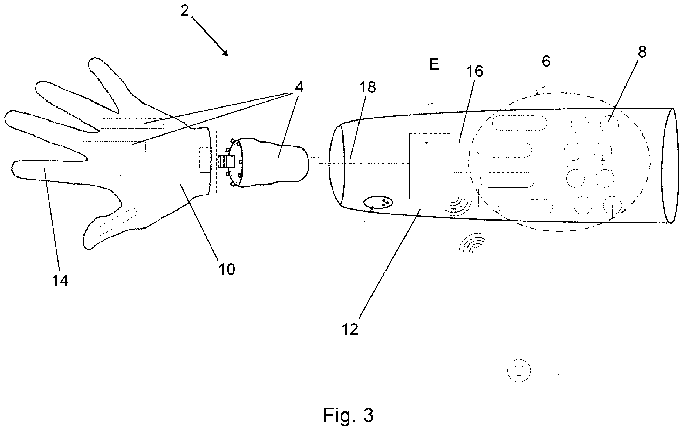

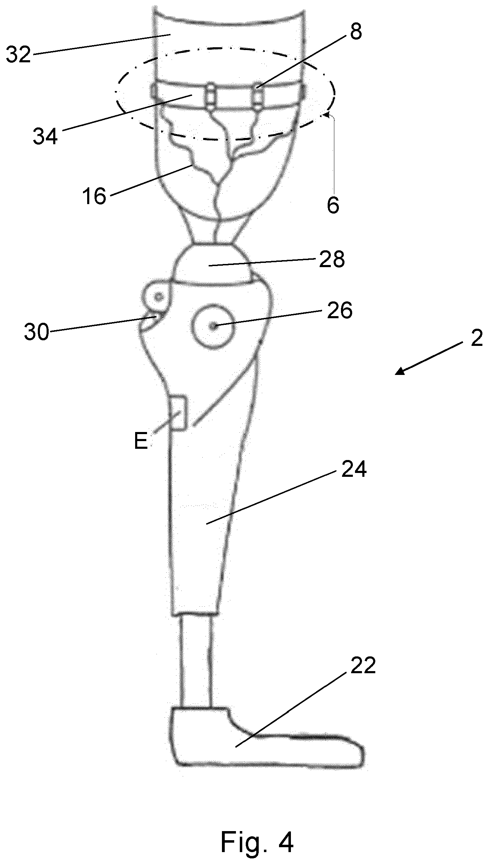

In Figur 2 ist ein schematisches Ablaufdiagramm dargestellt. Die zu steuernde und nicht in der Figur 2 , sondern in den Figuren 3 und 4 dargestellte Protheseneinrichtung 2 weist eine Sensoranordnung 6 mit acht Elektrodenpaaren 8 auf, was an den links angedeuteten acht Signalen zu erkennen ist. Diese Signale sind die Folge einer Bewegung und/oder einer Mehrzahl von Muskelkontraktionen eines Prothesenträgers. Diese werden an die elektronische Datenverarbeitungseinrichtung E geleitet und dort ausgewertet, also ggf. verstärkt, gefiltert und insbesondere mit in der elektronischen Datenverarbeitungseinrichtung E hinterlegten Körpersignalmustern abgeglichen. In der in Figur 2 dargestellten Ausführungsform sind zwei Sets, S1 und S2, in der elektronischen Datenverarbeitungseinrichtung E hinterlegt. In jedem Set sind jeweils drei Körpersignalmuster X, Y und Z hinterlegt. In dem ersten Set S1 sind den Körpersignalmustern X, Y, Z die Prothesenbewegungen 1 bis 3 zugeordnet. In dem zweiten Set S2 sind denselben Körpersignalmustern X, Y, Z die Prothesenbewegungen 4 bis 6 zugeordnet, die sich von den Prothesenbewegungen 1 bis 3 unterscheiden.

Es ist möglich, dass wie in Figur 2 alle Elektrodenpaare 8 Signale erfassen und an die elektronische Datenverarbeitung E leiten. Es ist jedoch ebenfalls möglich, dass für bestimmte Körpersignalmuster nicht alle Elektrodenpaare 8 ein Signal erfassen.

Hat die elektronische Datenverarbeitungseinrichtung E erkannt, dass z.B. das Körpersignalmuster X vorliegt, prüft die elektronische Datenverarbeitungseinrichtung E anschließend, welches der beiden Sets S1 oder S2 ausgewählt ist. Die Auswahl erfolgte zuvor beispielsweise durch Ausüben eines zugeordneten Triggersignals T.

Sofern das erste Set S1 ausgewählt ist, wird die Prothesenbewegung 1 aufgrund des erfassten Körpersignalmusters X ausgeführt. Sofern das zweite Set S2 ausgewählt ist, wird Prothesenbewegung 4 aufgrund des erfassten Körpersignalmusters X ausgeführt.

Die elektronische Datenverarbeitungseinrichtung E erzeugt dann vorzugsweise ein für die auszuführende Prothesenbewegung kodierendes Steuersignal und leitet dieses Steuersignal an den zumindest einen Antrieb 4 der Protheseneinrichtung 2. Der zumindest eine Antrieb 4 wird dann in Abhängigkeit des Steuersignals derart betrieben, dass die ausgewählte Prothesenbewegung, vorliegend 1 oder 4, je nachdem welches Set S1 oder S2 ausgewählt war, ausgeführt wird.

Figur 3 zeigt die schematische Darstellung einer Ausführungsform der aktuierbaren Protheseneinrichtung 2 einer oberen Extremität. Entlang ihrer Längsachse ist die Protheseneinrichtung 2 teilweise nach Art einer Explosionsdarstellung dargestellt. Die Protheseneinrichtung 2 ist als Prothesenunterarm ausgebildet und weist mehrere Antriebe 4, eine Sensoranordnung 6 mit einer Mehrzahl von Elektrodenpaaren 8 sowie eine elektronische Datenverarbeitungseinrichtung E auf.

Der in Figur 3 dargestellte rechte Antrieb 4 dient der Rotation einer Prothesenhand 10 der Protheseneinrichtung 2 relativ zu einem Unterarmschaft 12 der Protheseneinrichtung 2 um die Längsachse des Unterarmschaftes 12. Zusätzlich weist die Prothesenhand 10 eine Mehrzahl weiterer Antriebe 4 innerhalb der Prothesenhand 10 auf, die der Aktuierung der Prothesenfinger 14 der Prothesenhand 10 dienen.

Die Sensoranordnung 6 weist vier Elektrodenpaare 8 auf. Diese sind beispielsweise als einzelne Elektrodenpaare 8 ausgebildet, die jeweils auf der Haut eines Prothesenträgers befestigt werden können. Gemäß einer weiteren Ausführungsform sind die Elektrodenpaare 8 auf einem nicht dargestellten Prothesenliner aufgebracht, beispielsweise aufgeklebt oder integral mit dem Liner verbunden oder ausgebildet. Gemäß einer weiteren Ausführungsform sind die Elektrodenpaare 8 auf einer Innenseite des Unterarmschafts 12 angeordnet.

Die Elektrodenpaare 8 sind jeweils über Sensorleitungen 16 mit der elektronischen Datenverarbeitungseinrichtung E verbunden. Über die Sensorleitungen 16 werden die von den Elektroden erfassten Signale an die elektronische Datenverarbeitungseinrichtung E geleitet.

Die Signale werden in der elektronischen Datenverarbeitungseinrichtung E ausgewertet, insbesondere dahingehend, ob und welchem Körpersignalmuster die erhaltenen Signale entsprechend. Anschließend wird anhand des erfassten Körpersignalmusters und dem ausgewählten Set eine auszuführende Prothesenbewegung ermittelt. Ein für diese auszuführende Prothesenbewegung kodierendes Steuersignal wird dann mittels der Antriebsleitungen 18 an die Antriebe 4 geleitet.

In der in Figur 3 dargestellten Ausführungsform dient lediglich eine der schematisch dargestellten Antriebsleitungen 18 für den dargestellten Antrieb 4. Die anderen Antriebsleitungen 18 werden durch diesen hindurchgeführt oder sind an weitere Leitungen angeschlossen und versorgen die anderen Antriebe 4 in der Prothesenhand 10 zum Aktuieren der Fingerglieder 14.

Die elektronische Datenverarbeitungseinrichtung E steht vorliegend in drahtloser Verbindung, beispielsweise über Funk, mit einem Eingabegerät 20. Über dieses Eingabegerät 20 können beispielsweise erhaltene Signale der Sensoranordnung 6 visualisiert werden. Zudem ist es bevorzugt möglich, die in der elektronischen Datenverarbeitungseinrichtung E hinterlegten Sets und/oder Triggersignale zu verändern. Hierbei können beispielsweise Art und Anzahl der Körpersignalmuster und/oder Prothesenbewegungen verändert werden.

Das Eingabegerät 20 ist vorliegend ein Smartphone, insbesondere des Prothesenträgers. Hiermit kann er sich die erfassten Signale der Sensoranordnung 6 anzeigen lassen. Zusätzlich wird ihm vorzugsweise ein Soll-Wert, beispielsweise eine Soll-Intensität, des jeweiligen Signals angezeigt. Dadurch kann er erkennen, ob die Qualität seiner Ausführung ausreichend ist. Ist dies nicht der Fall, so kann er entweder trainieren oder die Soll-Werte werden an das tatsächlich Geleistete angepasst.

In Figur 4 ist eine weitere Ausführungsform der aktuierbaren Protheseneinrichtung 2, vorliegend in Form eines Prothesenbeines für einen oberschenkelamputierten Patienten. Die Protheseneinrichtung 2 weist eine Sensoranordnung 6 mit mehreren, z.B. sechs Elektrodenpaaren 8, von denen nur vier in Figur 4 sichtbar sind, auf.

Die Protheseneinrichtung 2 weist einen Prothesenfuß 22 und ein Unterschenkelteil 24 auf. An dem Unterschenkelteil 24 ist ein Prothesenkniegelenk 26 angeordnet, welches ein Gelenkoberteil 28 aufweist. Dem Prothesenkniegelenk 26 ist ein Dämpfer 30 oder Aktuator zugeordnet. An dem Gelenkoberteil 28 ist ein Prothesenschaft angeordnet, der der Übersichtlichkeit halber jedoch in Figur 4 nicht dargestellt ist. Stattdessen ist der darunterliegende Gliedmaßenstumpf 32 eines Prothesenträgers angedeutet.

Dem Prothesenkniegelenk 26 kann zudem ein nicht dargestellter Antrieb zugeordnet sein, der eine Flexions- oder Extensionsbewegung der Protheseneinrichtung 2 aktuiert. Diesem Antrieb 4 werden über ebenfalls nicht dargestellte Antriebsleitungen 18 Steuersignale von der elektronischen Datenverarbeitungseinrichtung E zugeleitet.

Die Sensoranordnung 6 weist einen flexiblen, insbesondere elastischen Gurt 34 auf, an dem die Elektrodenpaare 8 angeordnet sind. Dieser ist um den Gliedmaßenstumpf 34 herum angeordnet, sodass die Elektrodenpaare 8 an diesem anliegen und entsprechend Signale erfassen können. Von den Elektrodenpaaren 8 verlaufen jeweils Sensorleitungen 16 zu der elektronischen Datenverarbeitungseinrichtung E.

### Bezugszeichenliste

- 2: Aktuierbare Protheseneinrichtung
- 4: Antrieb
- 6: Sensoranordnung
- 8: Elektrodenpaar
- 10: Prothesenhand
- 12: Unterarmschaft
- 14: Prothesenfinger
- 16: Sensorleitung
- 18: Antriebsleitung
- 20: Eingabegerät
- 22: Prothesenfuß
- 24: Unterschenkelteil
- 26: Prothesenkniegelenk
- 28: Gelenkoberteil
- 30: Dämpfer
- 32: Gliedmaßenstumpf
- 34: Gurt

## Patentansprüche

1. Aktuierbare Protheseneinrichtung (2), mit
zumindest einem Antrieb (4),
einer elektronischen Datenverarbeitungseinrichtung (E), und
zumindest einer Sensoranordnung (6) zum Anordnen an einem Körperteil eines Prothesenträgers, wobei
die zumindest eine Sensoranordnung (6) eingerichtet ist zum Erfassen von Körpersignalmustern (X, Y, Z) und die elektronische Datenverarbeitungseinrichtung (E) eingerichtet ist, den zumindest einen Antrieb (4) in Abhängigkeit eines erfassten Körpersignalmusters (X, Y, Z) derart anzusteuern, dass die Protheseneinrichtung (2) eine dem Körpersignalmuster (X, Y, Z) zugeordnete Prothesenbewegung ausführt,
wobei in der elektronischen Datenverarbeitungseinrichtung (E) zumindest zwei Sets (S1, S2) aus Körpersignalmustern (X, Y, Z) und diesen jeweils zugeordneten Prothesenbewegungen hinterlegt sind, und
den Sets (S1, S2) zumindest ein Triggersignal (T) zugeordnet ist, das zwischen den Sets (S1, S2) umschaltet, **dadurch gekennzeichnet, dass** in mehreren Sets (S1, S2), bevorzugt in jedem Set (S1, S2), einem oder mehreren Körpersignalmustern (X, Y, Z) jeweils dieselben Prothesenbewegungen zugeordnet sind.

2. Aktuierbare Protheseneinrichtung (2) nach Anspruch 1, **dadurch gekennzeichnet, dass** in den Sets (S1, S2) zumindest ein Körpersignalmuster (X, Y, Z) identisch enthalten ist, wobei dem zumindest einen identisch enthaltenen Körpersignalmuster (X, Y, Z) in den Sets (S1, S2) jeweils unterschiedliche Prothesenbewegungen zugeordnet sind.

3. Aktuierbare Protheseneinrichtung (2) nach einem der Ansprüche 1 oder 2, **dadurch gekennzeichnet, dass** jedes Set (S1, S2) die identischen Körpersignalmuster (X, Y, Z) enthält.

4. Aktuierbare Protheseneinrichtung (2) nach einem der vorstehenden Ansprüche, **dadurch gekennzeichnet, dass** das zumindest eine Triggersignal (T) ein festgelegtes Körpersignal ist, das von den in den Sets (S1, S2) hinterlegten Körpersignalmustern (X, Y, Z) abweicht.

5. Aktuierbare Protheseneinrichtung (2) nach einem der vorstehenden Ansprüche, **dadurch gekennzeichnet, dass** jedes Set (S1, S2) zumindest vier Körpersignalmuster (X, Y, Z), insbesondere zumindest sechs Körpersignalmuster (X, Y, Z) umfasst.

6. Aktuierbare Protheseneinrichtung (2) nach einem der vorstehenden Ansprüche, **dadurch gekennzeichnet, dass** die Sensoranordnung (6) mehrere Elektrodenpaare (8) zum Erfassen der Körpersignalmuster (X, Y, Z) und insbesondere ein Elektrodenpaar (8) zum Erfassen des zumindest einen Triggersignals (T) aufweist.

7. Aktuierbare Protheseneinrichtung (2) nach einem der vorstehenden Ansprüche, **dadurch gekennzeichnet, dass** die Sets (S1, S2) mittels eines Eingabegeräts (20) veränderbar sind.

8. Elektronische Datenverarbeitungseinrichtung (E) für eine aktuierbare Protheseneinrichtung (2) nach einem der vorstehenden Ansprüche, wobei in der elektronischen Datenverarbeitungseinrichtung (E) zumindest zwei Sets (S1, S2) aus Körpersignalmustern (X, Y, Z) und diesen jeweils zugeordneten Prothesenbewegungen hinterlegt sind, und den Sets (S1, S2) zumindest ein Triggersignal (T) zugeordnet ist, das zwischen den Sets (S1, S2) umschaltet, wobei unabhängig von dem Set (S1, S2) zumindest einem Körpersignalmuster (X, Y, Z) dieselbe Prothesenbewegung zugeordnet ist.

9. Verfahren zum Aktuieren einer aktuierbaren Protheseneinrichtung (2) nach einem der Ansprüche 1 bis 7, mit den Schritten:
a) Auswählen eines anzuwendenden Sets (S1, S2) aus den zumindest zwei in der elektronischen Datenverarbeitungseinrichtung (E) hinterlegten Sets (S1, S2) durch Ausüben des zumindest einen Triggersignals (T),
b) Erfassen eines Körpersignalmusters (X, Y, Z) des Prothesenträgers mittels der zumindest einen Sensoranordnung (6), und
c) Aktuieren der Protheseneinrichtung (2) mittels des zumindest einen Antriebs (4) in Abhängigkeit des erfassten Körpersignalmusters (X, Y, Z) und des ausgewählten Sets (S1, S2), sodass die Protheseneinrichtung (2) die Prothesenbewegung ausführt, die dem erfassten Körpersignalmuster (X, Y, Z) in dem ausgewählten Set (S1, S2) zugeordnet ist, wobei unabhängig von dem ausgewählten Set (S1, S2) bei zumindest einem Körpersignalmuster (X, Y, Z) dieselbe Prothesenbewegung ausgeführt wird.

10. Verfahren nach Anspruch 9, **dadurch gekennzeichnet, dass** Schritt c) folgende Schritte aufweist:
c1) Leiten eines für das erfasste Körpersignalmuster (X, Y, Z) kodierenden Sensorsignals von der Sensoranordnung (6) zu der elektronischen Datenverarbeitungseinrichtung (E),
c2) Auswerten des Sensorsignals in der elektronischen Datenverarbeitungseinrichtung (E),
c3) Bestimmen einer auszuführenden Prothesenbewegung in Abhängigkeit des Sensorsignals und des ausgewählten Sets (S1, S2) und Erzeugen eines für die auszuführende Prothesenbewegung kodierenden Steuersignals,
c3) Leiten des Steuersignals an den zumindest einen Antrieb (4) der Protheseneinrichtung (2), und
c4) Betreiben des zumindest einen Antriebs (4) in Abhängigkeit des Steuersignals, sodass die Protheseneinrichtung (2) die auszuführende Prothesenbewegung ausführt.

## Claims

1. An actuatable prosthesis device (2), having
at least one drive (4),
an electronic data processing device (E), and
at least one sensor assembly (6) for arrangement on a body part of a prosthesis wearer, wherein
the at least one sensor assembly (6) is designed to detect body signal patterns (X, Y, Z), and the electronic data processing device (E) is designed to actuate the at least one drive (4) depending on a detected body signal pattern (X, Y, Z) in such a way that the prosthesis device (2) carries out a prosthesis movement assigned to the body signal pattern (X, Y, Z),
wherein at least two sets (S1, S2) of body signal patterns (X, Y, Z) and prosthesis movements assigned to each body signal pattern are stored on the electronic data processing device (E), and
at least one trigger signal (T) which switches over between the sets (S1, S2) is assigned to the sets (S1, S2), **characterized in that** in a plurality of sets (S1, S2), preferably in every set (S1, S2), one or more body signal patterns (X, Y, Z) are each assigned the same prosthesis movements.

2. The actuatable prosthesis device (2) as claimed in claim 1, **characterized in that** at least one body signal pattern (X, Y, Z) contained in the sets (S1, S2) is identical, wherein the at least one identical body signal pattern (X, Y, Z) contained in the sets (S1, S2) is assigned different prosthesis movements.

3. The actuatable prosthesis device (2) as claimed in either of claims 1 or 2, **characterized in that** each set (S1, S2) contains the identical body signal patterns (X, Y, Z).

4. The actuatable prosthesis device (2) as claimed in any one of the previous claims, **characterized in that** the at least one trigger signal (T) is a fixed body signal that differs from the body signal patterns (X, Y, Z) stored in the sets (S1, S2).

5. The actuatable prosthesis device (2) as claimed in any one of the previous claims, **characterized in that** each set (S1, S2) comprises at least four body signal patterns (X, Y, Z), in particular at least six body signal patterns (X, Y, Z).

6. The actuatable prosthesis device (2) as claimed in any one of the previous claims, **characterized in that** the sensor assembly (6) has a plurality of electrode pairs (8) for detecting the body signal patterns (X, Y, Z), and in particular one electrode pair (8) for detecting the at least one trigger signal (T).

7. The actuatable prosthesis device (2) as claimed in any one of the previous claims, **characterized in that** the sets (S1, S2) can be changed using an input device (20).

8. An electronic data processing device (E) for an actuatable prosthesis device (2) as claimed in one of the previous claims, wherein at least two sets (S1, S2) of body signal patterns (X, Y, Z) and prosthesis movements assigned to each body signal pattern are stored on the electronic data processing device (E), and the sets (S1, S2) are assigned at least one trigger signal (T) which switches over between the sets (S1, S2), wherein the same prosthesis movement is assigned to at least one body signal pattern (X, Y, Z) independently of the set (S1, S2).

9. A method for actuating an actuatable prosthesis device (2) as claimed in any one of claims 1 to 7, having the steps:
a) selecting a set (S1, S2) to be applied from the at least two sets (S1, S2) stored in the electronic data processing device (E) by issuing the at least one trigger signal (T),
b) detecting a body signal pattern (X, Y, Z) of the prosthesis wearer using the at least one sensor assembly (6), and
c) actuating the prosthesis device (2) by means of the at least one drive (4) depending on the detected body signal pattern (X, Y, Z) and the selected set (S1, S2), so that the prosthesis device (2) performs the prosthesis movement assigned to the detected body signal pattern (X, Y, Z) in the selected set (S1, S2), wherein the same prosthesis movement is assigned to at least one body signal pattern (X, Y, Z) independently of the set (S1, S2).

10. The method as claimed in claim 9, **characterized in that** step c) also comprises the following steps:
c1) routing a sensor signal coding for the detected body signal pattern (X, Y, Z) from the sensor assembly (6) to the electronic data processing device (E),
c2) evaluating the sensor signal in the electronic data processing device (E),
c3) determining a prosthesis movement to be performed, depending on the sensor signal and the selected set (S1, S2), and generating a control signal that codes for the prosthesis movement to be performed,
c3) routing the control signal to the at least one drive (4) of the prosthesis device (2), and
c4) operating the at least one drive (4) depending on the control signal so that the prosthesis device (2) performs the prosthesis movement to be performed.

## Revendications

1. Dispositif de prothèse actionnable (2), comprenant
au moins un entraînement (4),
un dispositif électronique de traitement de données (E), et
au moins une unité de détection (6) destinée à être disposée sur une partie du corps d'un porteur de prothèse,
dans lequel
ladite au moins une unité de détection (6) est conçue pour détecter des modèles de signaux corporels (X, Y, Z), et le dispositif électronique de traitement de données (E) est conçu pour commander ledit au moins un entraînement (4) en fonction d'un modèle de signal corporel (X, Y, Z) détecté, de telle sorte que le dispositif de prothèse (2) exécute un mouvement de prothèse associé au modèle de signal corporel (X, Y, Z),
au moins deux jeux (S1, S2) de modèles de signaux corporels (X, Y, Z) et de mouvements de prothèse respectivement associés à ceux-ci sont stockés dans le dispositif électronique de traitement de données (E), et
au moins un signal de déclenchement (T) est associé auxdits jeux (S1, S2), lequel commute entre les jeux (S1, S2),
**caractérisé en ce que**
les mêmes mouvement de prothèse respectifs sont associés à un ou à plusieurs modèles de signaux corporels (X, Y, Z) dans plusieurs jeux (S1, S2), de préférence dans chaque jeu (S1, S2).

2. Dispositif de prothèse actionnable (2) selon la revendication 1,
**caractérisé en ce qu'**au moins un modèle de signal corporel (X, Y, Z) est contenu de manière identique dans les jeux (S1, S2), des mouvements de prothèse respectifs différents étant associés audit au moins un modèle de signal corporel (X, Y, Z) contenu de manière identique dans les jeux (S1, S2).

3. Dispositif de prothèse actionnable (2) selon l'une des revendications 1 ou 2,
**caractérisé en ce que** chaque jeu (S1, S2) contient les modèles de signaux corporels identiques (X, Y, Z).

4. Dispositif de prothèse actionnable (2) selon l'une des revendications précédentes,
**caractérisé en ce que** ledit au moins un signal de déclenchement (T) est un signal corporel fixé qui diffère des modèles de signaux corporels (X, Y, Z) stockés dans les jeux (S1, S2).

5. Dispositif de prothèse actionnable (2) selon l'une des revendications précédentes,
**caractérisé en ce que** chaque jeu (S1, S2) comprend au moins quatre modèles de signaux corporels (X, Y, Z), en particulier au moins six modèles de signaux corporels (X, Y, Z).

6. Dispositif de prothèse actionnable (2) selon l'une des revendications précédentes,
**caractérisé en ce que** l'unité de détection (6) comprend plusieurs paires d'électrodes (8) destinées à détecter les modèles de signaux corporels (X, Y, Z) et en particulier une paire d'électrodes (8) destinées à détecter ledit au moins un signal de déclenchement (T).

7. Dispositif de prothèse actionnable (2) selon l'une des revendications précédentes,
**caractérisé en ce que** les jeux (S1, S2) peuvent être modifiés au moyen d'un appareil d'entrée (20).

8. Dispositif électronique de traitement de données (E) pour un dispositif de prothèse actionnable (2) selon l'une des revendications précédentes,
dans lequel au moins deux jeux (S1, S2) de modèles de signaux corporels (X, Y, Z) et de mouvements de prothèse respectivement associés à ceux-ci sont stockés dans le dispositif électronique de traitement de données (E), et au moins un signal de déclenchement (T) est associé aux jeux (S1, S2), lequel commute entre les jeux (S1, S2), et le même mouvement de prothèse est associé à au moins un modèle de signal corporel (X, Y, Z) indépendamment du jeu (S1, S2).

9. Procédé d'actionnement d'un dispositif de prothèse actionnable (2) selon l'une des revendications 1 à 7,
comprenant les étapes consistant à :
a) sélectionner un jeu à appliquer (S1, S2) parmi lesdits au moins deux jeux (S1, S2) stockés dans le dispositif électronique de traitement de données (E), en appliquant ledit au moins un signal de déclenchement (T),
b) détecter un modèle de signal corporel (X, Y, Z) du porteur de la prothèse au moyen de ladite au moins une unité de détection (6), et
c) actionner le dispositif de prothèse (2) au moyen dudit au moins un entraînement (4) en fonction du modèle de signal corporel (X, Y, Z) détecté et du jeu (S1, S2) sélectionné, de sorte que le dispositif de prothèse (2) exécute le mouvement de prothèse associé au modèle de signal corporel détecté (X, Y, Z) dans le jeu (S1, S2) sélectionné, sachant que le même mouvement de prothèse est exécuté pour au moins un modèle de signal corporel, indépendamment du jeu (S1, S2) sélectionné.

10. Procédé selon la revendication 9,
**caractérisé en ce que** l'étape c) comprend les étapes suivantes consistant à :
c1) transmettre un signal de détection, encodant le modèle de signal corporel (X, Y, Z) détecté, de l'unité de détection (6) au dispositif électronique de traitement de données (E),
c2) évaluer le signal de détection dans le dispositif électronique de traitement de données (E),
c3) déterminer un mouvement de prothèse à exécuter en fonction du signal de détection et du jeu (S1, S2) sélectionné, et générer un signal de commande encodant le mouvement de prothèse à exécuter,
c3) transmettre le signal de commande audit au moins un entraînement (4) du dispositif de prothèse (2), et
c4) faire fonctionner ledit au moins un entraînement (4) en fonction du signal de commande, de sorte que le dispositif de prothèse (2) exécute le mouvement de prothèse à exécuter.
